# EUROPEAN PATENT APPLICATION

(11) **EP 4 342 998 A1**
(43) Date of publication of application: **27.03.2024**
(21) Application number: 22197285.4
(22) Date of filing: 23.09.2022
(51) Int. Cl.: C12Q 1/6816

(54) **LABELED PROBES WITH DIFFERENTIALLY CLEAVABLE LINKERS AND THEIR USE IN DE-CODING DNA AND RNA MOLECULES**

(71) Applicant: Miltenyi Biotec B.V. & Co. KG, 51429 Bergisch Gladbach (DE)
(72) Inventor: MARMA, Mong, 51429 Bergisch Gladbach (DE); PERBOST, Michel, 51429 Bergisch Gladbach (DE); PINARD, Robert, 51429 Bergisch Gladbach (DE); GUAN, Xiao-Pei, 51429 Bergisch Gladbach (DE); SOLIMAN, Sameh, 51429 Bergisch Gladbach (DE); NEIL, Emily, 51429 Bergisch Gladbach (DE)
(74) Representative: Kisters, Michael Marcus

(57) **Abstract**

The invention is directed to a method for detecting RNA, DNA or protein target sequences by
a) Hybridizing a library of probes having the general formula (I)

P - (CL-D)ₓ (I)

With
P: probes having at least 10 nucleotides or amino acids
CL: cleavable linker
D: fluorescent dye
X: integer between 1 and 5

to RNA, DNA or protein target sequences wherein the library comprises probes P having different sequences of nucleotides or amino acids and cleavable linkers CL of different groups which are cleavable with different means

b) Removing unhybridized probes and detecting the hybridized probes *via* the fluorophores D by a first image
c) Cleaving sequentially by different means each group of chemical linkers CL from the hybridized probes; removing the thus cleaved fluorophores D and detecting the remaining hybridized probes via their fluorophores D by a second image
d) Detecting the removed fluorophores D by comparing the first and second image.
e) Obtaining a part of the sequence information of the target sequences via the sequence information of the probes P associated with the removed fluorophores D
f) Repeating step c) until all groups of chemical linkers CL are cleaved.

## Description

### BACKGROUND

The present invention is directed to a new way of de-coding DNA and RNA molecules, by hybridizing labeled DNA probes, which are conjugated to fluorescent labels or tags *via* cleavable linkers (CL). A library of DNA probes that are conjugated to labels by different linkers, which can be cleaved off selectively at different conditions and at predictable fashion.

Labeled probes have many applications in modern biology and medicines. For example, labeled DNA probes are used for detecting specific target sequence of DNA or RNA by hybridization. They are the basis of DNA microarray technologies, in-situ detection of specific sequence of DNA or RNA molecules (e.g. FISH), and in DNA sequencing by ligation, to name a few. The same way, labeled antibody are used to detect specific antigen targets.

Traditionally, labeled DNA probes with fluorescent reporter units are attached to probes through stable linkers. When such labeled probes are used, they can be removed only under de-hybridizing conditions such as at higher temperature, low salt, use of formamide, high pH, etc. For probing multiple targets, this method requires multiple rounds of hybridization and de-hybridization steps. Both steps are relatively slow: typically each step requires between half an hour to overnight reaction depending on the complexity and length of the probes.

Further, the relatively harsh conditions used to perform these steps can be damaging to nucleic acid targets and to the substrate on which the target DNA or RNA molecules are fixed, such as tissue, surface coating etc. Therefore, they can limit how many number of iterative cycles of hybridization - de-hydridization steps can be performed. In this case, the throughput depends on the number of dyes are used, and the number of rounds of hybridization and de-hybridization steps employed. Both can be limiting factors. The same issues persists in detecting other biomolecules such as of antibody, cellular carbohydrates and peptides.

Accordingly, the objective of the current invention is to provide a fast and high throughput process for de-coding target DNA and RNA molecules with minimum repeating of hybridization and de-hybridization rounds.

Current state of the art in RNA sequencing by hybridization (SBS) technology involves the use of labeled DNA probes with non-cleavable linker and/or use of indiscriminately cleavable disulfide linker. Therefore, current methods do not allow stepwise cleavage and pattern generation by sequential cleave step for increased throughput of de-coding. Those methods rely solely on the de-hybridization or single step cleavage for removing fluorescent reporters from the targets. Therefore, they naturally have limited throughput and applications. These methods may not be compatible with certain surface chemistries or matrixes such as when used in in-situ sequencing and spatial transcriptomics studies due to degradation of the tissue due to repeated treatment with de-hybridization steps.

For example, WO2015054050A1 discloses a method for probing multiple target wherein the probes are cleaved, but each cleaving step is performed in the same way. Accordingly, the cleaving step is not used to differentiate between different probes.

### SUMMARY

It was found that when a library of labeled probes comprised of different cleavable linkers between the probe and the label, a sequential removal of the labeling molecules can be achieved in a predictable and controllable fashion.

Object of the invention is therefore a method for detecting RNA, DNA or protein target sequences by
a) Hybridizing a library of probes having the general formula (I)

   P - (CL-D)ₓ (I)

   With
      P: probes having at least 10 nucleotides or amino acids
      CL: cleavable linker
      D: fluorescent dye
      X: integer between 1 and 5
   to RNA, DNA or protein target sequences wherein the library comprises probes P having different sequences of nucleotides or amino acids and cleavable linkers CL of different groups which are cleavable with different means
b) Removing unhybridized probes and detecting the hybridized probes *via* the fluorophores D by a first image
c) Cleaving sequentially by different means each group of chemical linkers CL from the hybridized probes; removing the thus cleaved fluorophores D and detecting the remaining hybridized probes via their fluorophores D by a second image
d) Detecting the removed fluorophores D by comparing the first and second image.
e) Obtaining a part of the sequence information of the target sequences via the sequence information of the probes P associated with the removed fluorophores D
f) Repeating step c) until all groups of chemical linkers CL are cleaved.

An important feature of the method of the present invention is the use of a library of labeled probes where the reporter molecules or tags are attached via sets or groups of cleavable linkers (CL) that allow fast cleavage at different conditions - by chemical, photochemical, electrochemical, and/or enzymatic means. Since cleavage chemistry is much faster that de-hybridizing DNA probes, this method is an intrinsically faster way of de-coding the RNA or DNA targets.

By sequential removal of the labels, target sequences can be deciphered by comparing fluorescence signatures before and after the individual cleave steps. The main advantage of this method is its ability to de-code greater number of DNA and RNA targets in each round of hybridization. And by combining iterative cycles of hybridization and sequential cleavage steps, and by re-hybridization with a new set of probes, a large number of target genes can be decoded.

This new class of DNA probes is referred to as differentially cleavable linker - DNA probes: DCL-DNA probes. In the method of the invention, a library of DCL-DNA probes is hybridized for sequence specific binding to the targets. After initial fluorescence imaging, the samples are treated with different cleaving reagents in a sequence. After each round of cleaving treatment, further fluorescence images are taken. From the change of fluorescent signal, before and after the cleaving steps, which cause certain fluorescent signals to get extinguished, the target sequences are de-coded. In the conventional methods, the number of targets that can be detected is dependent on the number of fluorescent dyes. In the method of the invention, multiple targets can be detected from each individual dyes due to coupling to different cleavable linkers and to their differential response to cleave reagents. A greatly higher number of targets could be detected with limited number of dyes when this new method used compared to traditional hybridization method.

### DESCRIPTION OF THE DRAWINGS

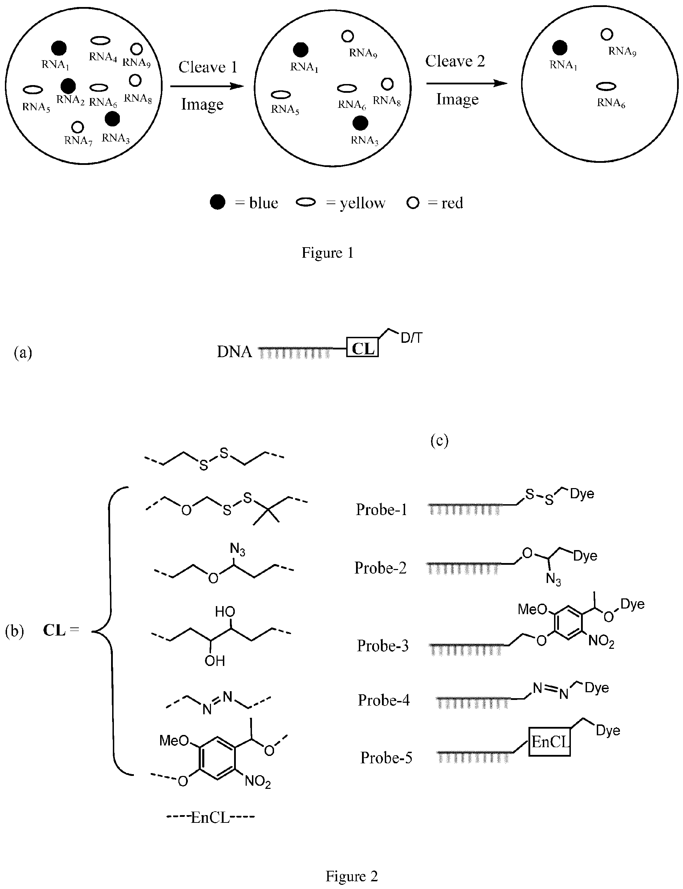

Figure 1 shows a schematic presentation how 9 RNA targets can be detected by a single event of hybridization using DNA barcodes labeled with only three different dyes (blue, yellow and red) via differently cleavable linkers. In the methods of the prior art, only three targets can be distinguished with three dyes. In this new method, although RNA₁, RNA₂ and RNA₃ fluoresce in the same blue channel, they are distinguished from each other due to their response to cleave reagents. RNA₂ and RNA₃ fluorescence get extinguished when treated with cleave reagent 1 and 2, respectively. On the other hand, RNA₁ was not impacted due to coupling to dye by non-cleavable linker (NC). The same way other RNA targets (RNA₄₋₉) can be detected in yellow and red channels.
Figure 2(a) shows a generic structure of DCL-DNA probes, CL = cleavable linker, EnCL = enzymatically cleavable linker. D/T = fluorescent dyes or molecular tags. Figure 2(b) shows some of the cleavable functional groups, e.g. disulfide (-S-S-), oxymethylene disulfide (-OCH₂-SS-), azidomethine (-OCH-N₃-), azoarene (-ArN=NAr-), dial (-CH(OH)-CH(OH)-), photocleavable-nitrobenzyl and enzymatically cleavable linkers. Figure 2(c) shows some representative structures of DLC-DNA probes with cleavable linkers. It also shows how multiple DNA probes can be labeled by single fluorescent dyes *via* different cleavable linkers.
Figure 3 (a) shows schematic representation of differentially cleavable linked labeled DNA probes library sets (DCL-DNA Probe sets) than can be created by combining various cleavable linkers and labeling by one or more labels. Figure 3(b) shows some representative schematic structures.
Figure 4 shows how 24 targets can be detected by single step hybridization with DCL-DNA probes using only three fluorescent dyes. By conventional methods, only three targets can be probed by three dyes labeled DNA probes, not 24 targets.
Figure 5 shows partial structures of two reversible nucleotide terminators which could be used after DNA probe hybridization method of de-coding further sequence the targets. Combination of hybridization and sequencing by synthesis (SBS) chemistry can dramatically increase the throughput of target sequence detection. In this figure, CL stands for a cleavable linker and the 3'-OH capping groups are -CH₂SSMe and -CH₂N₃.

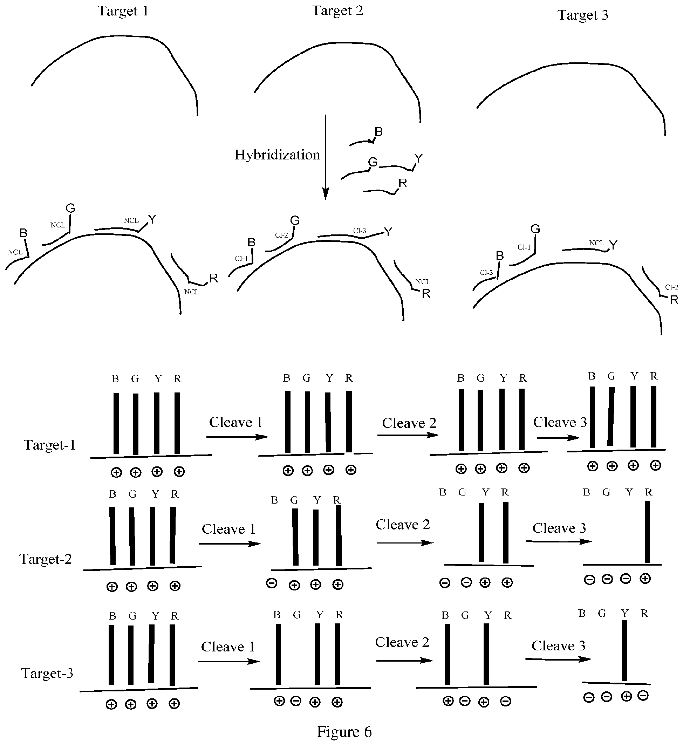

Figure 6 shows how three DNA or RNA targets can be de-coded by a single event of hybridization, in four color fashion using four fluorescent dyes labeled DNA probes. Target-1 retains fluorescent signals with three different cleave conditions, because all bonded probes are non-cleavable linker (NC) linked DNA Probes. Target-2 and target -3 loses three dyes sequentially upon cleavage steps (three probes are CL linked and one is NC linked labeled DNA probes), but in different patterns after treating with cleave reagents. From the color patterns all three targets can be identified. In this figure, B = blue dye, G = green dye, Y = yellow dye, R = red dye.

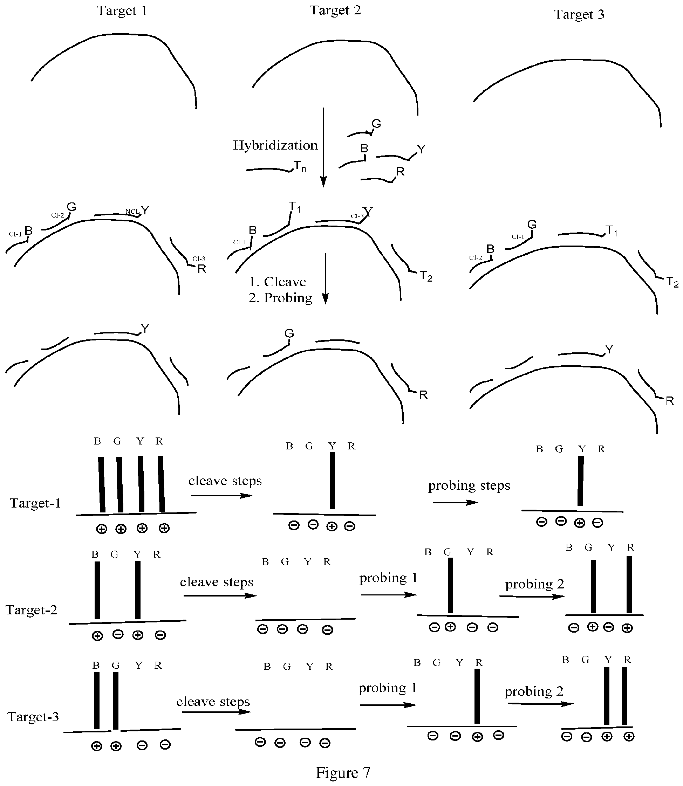

Figure 7 shows how a combination of labeled DCL-DNA probes and non-cleavable labeled DNA probes with tags can be used to de-code multiple targets by single event of hybridization. In target-1, all fluorescent dyes get removed except one upon cleavage steps, and probing it does not cause any re-appearance of the other fluorescent signatures. On the other hand, the target-2 and target-3 fluorescent dyes get removed after treating with cleave reagents (all probes are DCL- DNA Probes), but re-appear the fluorescent signatures after probing the tag, but in different fashion, target-2 re-appears green and red, while target-3 re-appears yellow and red colors. By using various combination and cleavable, non-cleavable and tags labeled DNA probes a large number of target genes could be decoded. In this figure B = blue dye, G = green dye, Y = yellow dye, R = red dye.
Figure 8 shows the chemical reaction steps for the synthesis of DCL-DNA probes with oxymethylene disulfide (-OCH₂SS-) linker, which can be cleaved off effectively by phosphine and thiol based cleave reagents (e.g. DMPS).
Figure 9 shows the chemical reactions steps for the synthesis DCL-DNA probes with azidomethine (-OCH-N₃-) cleavable linker.
Figure 10 shows probes suitable for the method of the invention
Figure 11 shows the general workflow of the invention
Figure 12 and 13 show staining results

### DETAILED DESCRIPTION

In the methods of the prior art for de-coding DNA and RNA targets, such as seq-FISH, MERSISH method etc, by using fluorescently labeled DNA barcodes can sequence a maximum of F^{N} targets, where F = number of fluorophores, N = number of hybridization steps. With 4 fluorophores and 5 rounds of hybridization only 4⁵ =1,024 targets can be detected.

On the other hand, with the method of the present invention, with the same number of fluorescent dyes and a library of DCL-DNA probes with 4 sequentially cleavable linkers (CL), potentially (F × CL)^{N} = (4 × 4)⁵ = 1,048,576 targets can be detected, where F = number of fluorophores, N = number of hybridization steps, CL = number of differently cleavable linkers.

Not only that, DCL-DNA probes can carry single or multiple fluorescence reporters which can be removed under the same or different conditions. Further, it can be used in combination with tagged DNA probes, e.g. biotin, hapten, or antigen labels etc. which can be probed by secondary interaction. Given the number of combinations, the number of detectable targets can go up dramatically. Furthermore, this hybridization method, optionally with or without de-hydridization step needed, could speeds up the de-coding process.

Furthermore, due to the rounds in the method of the invention comprising hybridization and sequential cleave, the detection and rehybridization steps cover a plurality of probes, which exponentially multiply the de-coding power of this method. The method can sequence exceedingly a large number of DNA and RNA targets, unlike other traditional methods.

One specific example, but not limited to, is the use for Fluorescence In-Situ Hybridization (FISH) methods for sequencing RNA transcripts and in spatial transcriptomics studies. Also, the same cleavable linkers can be used to detect other biomolecules such as proteins, antibody, antigens, carbohydrates, etc for multi-omics applications.

The present invention provides a faster way of detecting nucleic acid sequences by using a minimum number of hybridization steps. The sample is imaged after hybridization and in sequence after each cleavage reaction to generate differential patterns for targets, which can be used to decode nucleic acid targets. A general scheme is shown in Figure 1, wherein multiple RNA targets are hybridized with the same color probes (3 blue: RNA₁₋₃, 3 red: RNA₄₋₆, 3 yellow: RNA₇₋₉) but with different cleavable linkers. RNA₁, RNA₂ and RNA₃ have the same blue dye but they response differently to different cleave reagents - RNA₁ stays the same, but RNA₂ and RNA₃ get extinguished at different stages of cleave treatments. The same for other RNA targets. The response to the cleave reagents allows reading those RNA targets despite having the same dye. If there were no differently cleavable linkers, then only three RNA targets could be decoded for three dyes, not nine targets.

With current state of arts, when 4 color labeled DNA probes are used, a single event of hybridization can provide only 16 decoding possibilities even when multiple combination of dyes are used: 1 code with all 4 colors, 4 codes with 3 colors, 6 for 2 colors, 4 with 1 and finally 1 with no colors.

On the other hand, with 4 color and 4 differently cleavable linkers, the present DCL-DNA probes system, a single event of hybridization with all possible probes together, and subsequent 4 selectively cleavage steps and molecular probing can lead to decoding capacity goes up from 16 (as discussed above) to more than 500 possibilities.

Figure 2 shows a schematic representation of labeled DNA probes with differentially cleavable linker (DCL-DNA Probes). It also shows some representative structures of cleavable linkers (Fig 2(b)) and how cleavable linkers can be used to create a set of labeled DNA probes from a single fluorescent dye (Fig 2 (c)) with a set of variable linkers.

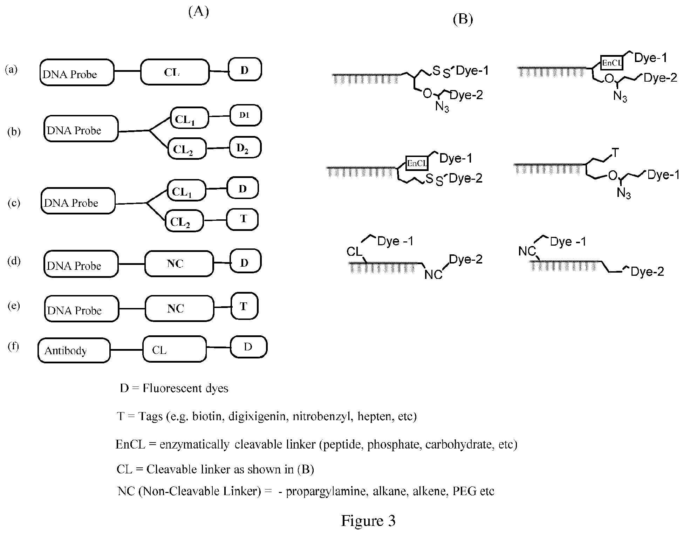

Figure 3 shows an example, but not limited to, how a large library set of DCL-DNA probes can be built using various combination of cleavable (CL), non-cleavable (NC), molecular tags (T) and labeling by multiple reporters on DNA backbones.

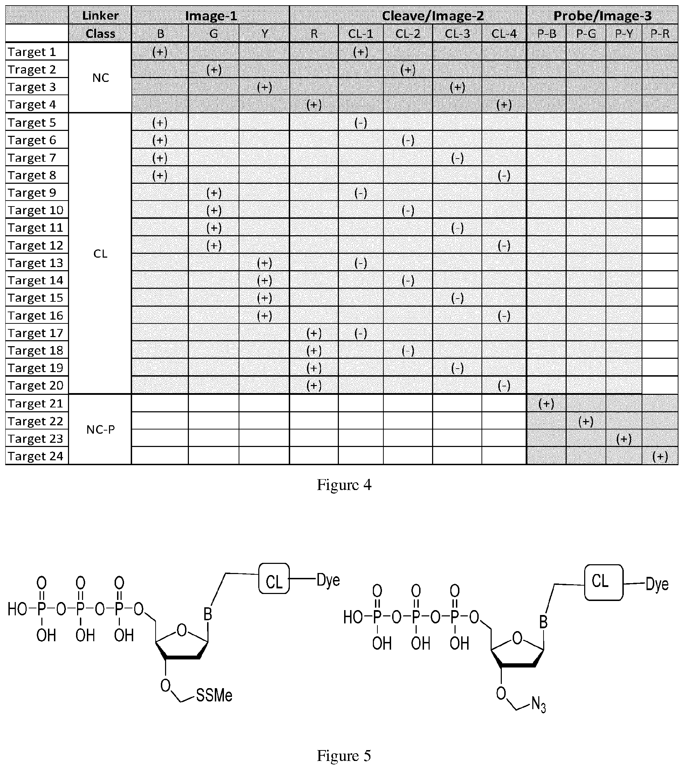

Figure 4 shows how a single hybridization event can read over 20 targets using only 4 dyes. In methods of the prior arts only 4 targets can be detected with single dye labeled probes from 4 dyes. On the other hand, by using differently cleavable linkers, each dye can detect multiple targets. In this figure, target 5 to 8, though labeled by the same blue dye labels, are distinguished due to different response to different cleave reagents (in this case, dyes are extinguished at different cleave conditions). Therefore, the current invention helps to increase each fluorescent dye to multiple discernable dyes. For example, one single blue dye can be used to distinguish 7 different targets by using 7 differently cleavable linkers.

In the method according to the invention, after step f), the probes P are optionally de-hybridized from the RNA, DNA or protein target sequences.

Further, step a) to f) can be repeated until the sequence information of the RNA, DNA or protein target sequences is obtained. Obtaining the sequence information of the RNA, DNA or protein target sequences includes fully (complete, 100%) sequence information as well as partially information. The amount of information and in turn the speed of the method may be controlled though the selection of probes i.e. the selection of nucleotides or proteins of the probes. Obtaining information may be in the range of 10 - 100%, although a more or less full sequence analyses is preferred (like 90-100%).

The hybridization based method of the invention with cleavable linker can further be combined with sequencing by synthesis chemistry (SBS chemistry). SBS chemistry can be started after reading with DCL-DNA probes and cleaving off the fluorescent dyes. In this case, the DNA probes can be used as sequencing primers without needing to remove from the targets. SBS chemistry relies on the use of reversibly terminating nucleotides with fluorescent dye labels via cleavable linker. Two representative reversibly terminating nucleotides that can be used in combination with DCL-DNA probes are shown in figure 5, where CL stands for a chemically cleavable linker that link fluorescent dyes to the nucleobases and the capping group on 3'-OH is preferable -CH₂SSMe or -CH₂N₃. The cleavable linkers can be the same as DCL linker or different.

The DCL-DNA probes can also be used along with DNA probes with non-cleavable reporter groups and attached molecular tags. The tags allow probing the target by secondary interaction. In that situation, the non-cleavable, labeled DNA probes can be used not only for reading specific target sequences but also as an internal control. The tags can be for example biotic, digitogenin, antigen, hapten etc.

The DNA probes may contain a single or multiple fluorescent dye with the same cleavable linker or different cleavable linker. The may also contain combination of fluorescent dyes and tags. The tags can be probed by secondary interaction, such as DNA-biotin probe detected by interaction with labeled streptavidin, etc.

The terms "differently cleavable linkers", "different groups of linkers", "multiplicity of linkers" are interchangeable and refer to sets of linkers which are cleavable under different conditions which allow removal of the label in sequence in a controllable and predictable fashion.

The term "cleavable linkers CL which are cleavable with different means" refers to cleavable linkers CL which are cleavable with a first method, but not with a second. The different means or method for cleaving enables sequentially taking images of different group of probes staining the target.

In the method of the invention, chemical, photochemical or enzymatical means for cleaving the cleavable linkers CL may be used. For example, the library comprises probes having at least two different groups of cleavable linkers CL which are cleavable with two different means selected from the group consisting of two different photochemical activation radiations, two different enzymes, two different chemical agents, one photochemical activation radiation and one enzyme, one photochemical activation radiation and one chemical agent, one enzyme and one chemical agent.

Of course, the cleavable linkers CL may be cleavable with three, four of five different means without deviating from the gist of the invention, For example it is possible to utilize two different enzymes and two different chemical agents or four different chemical agents to obtain 4 different de-staining images.

The cleavable linker CL may be selected from the group consisting of disulfide linkers (-SS-, (a)), oxymethylene disulfides (-OCH2SSCR1R2-, (b)), oxymethine-azides (-CR1,R2OCH(N3)R3,R4)-, (c)), azo-arenes (-ArN=NAr-, (d)), nitrobenzyl derivatives - (e), allyl derivatives (f), thiocarbamate (g), where R1, R2, R3, R4 can be independently H, methyl, ethyl, propyl, t-butyl, C5-C10 alkyls, alkenes, alkynes, hydroxyl, halogens, amines, amides, carboxylates, polyethylene glycols, for example as shown in the following formulas a) to g).

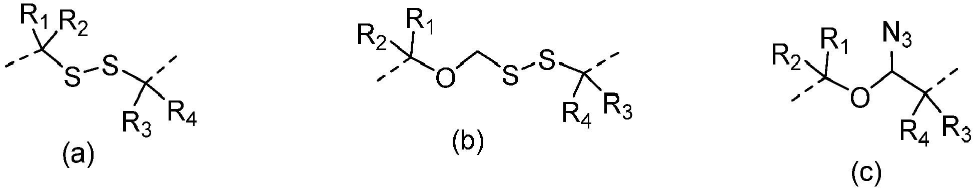

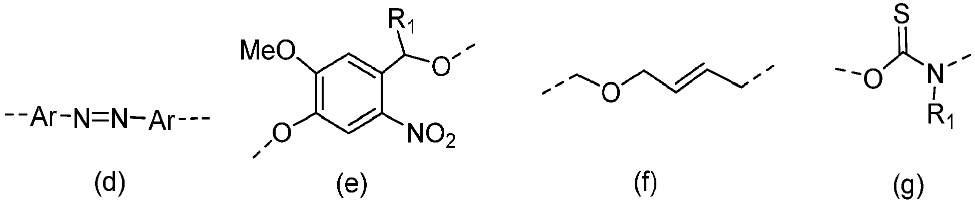

The cleaving reaction/chemistry depends on the cleavable linker (CL) and may be chosen as appropriate from the following:

Disulfide linkers can be cleaved off selectively by treating with thiols or phosphines leaving other linkers intact.

Oxymethine-azide (-OCH(N₃)-) can be selectively cleaved off in the presence of azo-arenes, photocleavable or other enzymatically cleavable linkers when treated with phosphines (e.g. TCEP).

Alternatively, TCEP can be used to cleave off both disulfide and oxymethine azide linkers. Further, azo-arene linker can be cleaved off selectively with very mild reagent - sodium dithinite (Na₂S₂SO₄) in aqueous solution buffer.

Cleave reagents can be thiols (e.g. dithiothreitol -DTT, dimercapto-propane sulfonates - DMPS, etc), phosphines (e.g. TCEP, THPP, etc), mild reducing agents (e.g. Na₂S₂O₄), specific wavelength of light, electric potential, enzymes (e.g. peptidase, dextranase, esterase, phosphatase, proteinase etc).

Enzymatically cleavable linkers can be any molecule which can be cleaved off or digested by a specific enzyme like peptidase. Suitable as enzymatically degradable linkers are, for example, polysaccharides, proteins, peptides, depsipeptides, polyesters, nucleic acids, and derivatives thereof. Suitable polysaccharides are, for example, dextrans, pullulans, inulins, amylose, cellulose, hemicelluloses, such as xylan or glucomannan, pectin, chitosan, or chitin. They may be derivatized to provide functional groups for covalent or non-covalent binding of the linker. Proteins, peptides, and depsipeptides used as enzymatically degradable linker can be functionalized via side chain functional groups of amino acids. Polyesters, polyacrylamide and polyesteramides used as enzymatically degradable linker can either be synthesized with co-monomers, which provide side chain functionality or be subsequently functionalized. In the case of branched polyesters functionalization can be via the carboxyl, amine or hydroxyl end groups. Post polymerization functionalization of the polymer chain can be, for example, via addition to unsaturated bonds, i.e. thiolene reactions, or they can be done by azide-alkyne or tetrazine alkyne click reactions, or via introduction of functional groups by radical reactions.

The enzymatically degradable linker is degraded by the addition of an appropriate enzyme. The choice of enzyme as cleave reagent is determined by the chemical nature of the enzymatically degradable linker and can be one or a mixture of different enzymes. Such enzymes can hydrolases, lyases, reductases, esterase, peptidase etc. Preferable enzymes may be is selected from the group consisting of glycosidases, dextranases, pullulanases, amylases, inulinases, cellulases, hemicellulases, pectinases, chitosanases, chitinases, proteinases, esterases, glycosidase, pyrophosphatase, lipases, phosphatase, and nucleases.

The method of the invention can be used with multi-probe hybridization method, where more than one probe binds to a specific target. Figure 6 shows how three DNA targets can be de-coded by a single event of hybridization and sequential cleavage and imaging steps, in a four-color fashion using four fluorescent dyes labeled DNA probes. The calling of specific genes is obtained from the fluorescent response to cleave reagents, and by comparing the initial images with that of the a subsequent steps fluorescence due to exposure to cleave reagents. Target-1 retains fluorescent signals with three different cleave conditions, because all are non-cleavable linker (NC) linked DNA Probes. Target-2 and target -3 loses three dyes sequentially upon cleavage reactions but in different pattern.

In another aspect of the invention, the DNA probes may contain more than one fluorescent dye or tag, or combination thereof (Figure 7). This construct will allow selectively cleaving off of the dyes of interest and probing the target in later step by secondary interaction with tags. The use of such combination is shown in figure 7. Its shows how three different targets can be distinguished by probing with a mixture comprising of DCL and non-cleavable (NC) - tags linked labeled DNA probes. The three targets are responding differently to cleave and molecular probing on the tags. Target-1 loses all fluorescent signals except one upon treatment of cleave reagent and does not response to molecular probing. On the other hands, target 2 and target 3 lose all fluorescent signatures and responded differently to later probing to attached tags. By using various combination and cleavable, non-cleavable and Tags labeled DNA probes a large number of target genes could be decoded.

In another aspect of the invention, after removing the fluorescent dyes, the free 3'-OH of the probe DNA can be used for single base extension and running additional sequencing by synthesis chemistry by using labeled reversibly terminating nucleotides. Reversible terminator nucleotides stop complementary strand synthesis by single base extension, but can be further extended after removing the 3'-OH capping group. The reversibly terminating nucleotides can be 3'-OH capped with methyl methylene disulfide (-CH2SSMe), or azidomethyl (-CH₂N₃) with the generic structures shown in figure 4. These nucleotides may contain DCL linkers as in DNA probe design. They may be used independently or in combination of with DCL-Probes.

In another aspect of the invention, the hybridization involves the use of different probes with their own specific targets and involves multicolor fluorescence measurement. It can also involve more than one cycle of cleave and image steps in each hybridization event, and it can also involve one or more steps of hybridization - imaging - cleaving - imaging and re-hybridization cycle with optional de-hybridization step between each cycle. It can also involve one or more steps of probing by secondary interaction, two specific examples but not limited to, are biotin- streptavidin, DIG - anti-DIG interaction and imaging afterwards.

### Probes, Labels and Tags

The probes can be regular, natural or non-natural DNA or RNAs and their modified analogues, i.e. consist of natural or non-natural nucleotides. They can be proteins too, i.e. consist of natural or non-natural amino acids. The DNA probes can be locked nucleic acids which provides better duplex stability. They can have modification on 3' or 5' end, or modification can be on any of the bases on the main backbone, such as dUTP with amino modification. The label can be fluorescent molecules (rhodhamine dyes such (R₆G, ROX), cyanine dyes (Cy3, Cy5), Alexa dyes, ATTO dyes, etc) or can be energy transfer dyes or quencher. The molecular tags can be biotin, happens, antigens, DIG, fluorescein, nitrophenyl etc, or any modification that be identified by secondary interaction (e.g. biotin- STV, antibody-antigen, DIG- anti-DIG interaction with fluorescent labeling). DCL linkers can be on probing proteins too.

### Target Sequences and Method

Target sequences can be DNA or RNA, including mRNA. It can be used in a Florescence In-Situ Hybridization (FISH) or sequential FISH (seqFISH) method. The decoding can be on clonally amplified - DNA clones on beads, surface bound rolling cycle amplified (RCA) products or on tissues, or single molecule, or spatially resolved single cells. They can be used in-situ or ex-situ sequencing. The decoding method can involve multiple rounds of hybridization steps to increase the decoding capacity. The de-coding method can involve hybridization with mixture of labeled DNA probes followed by imaging, sequential cleavage steps and imaging, and probing the molecular tags by secondary interactions. Targets can be proteins, peptide, carbohydrate, antibody, and other biomolecules.

### Fluorescent dyes

Suitable fluorescent dyes are those known from the art of fluorescence technologies, e.g., flow cytometry or fluorescence microscopy. For example, fluorescent dyes are xanthene dyes, like fluorescein, or rhodamine dyes, coumarine dyes, cyanine dyes, pyrene dyes, oxazine dyes, pyridyl oxazole dyes, cascade dyes, polymeric dyes, pyromethene dyes, acridine dyes, oxadiazole dyes, carbopyronine dyes, benzpyrylium dyes, fluorene dyes, or metallo-organic complexes, such as Ru, Eu, Pt complexes. Besides single molecule entities, clusters of small organic molecule dyes, fluorescent oligomers or fluorescent polymers, such as polyfluorene, can also be used as fluorescent moieties. Additionally, fluorescent dyes might be protein-based, such as phycobiliproteins, nanoparticles, such as quantum dots, upconverting nanoparticles, gold nanoparticles, dyed polymer nanoparticles.

### EXAMPLES

To establish a proof-of-concept on the use of DCL-DNA probes for mRNA sequencing, multiple sets of probes were synthesized. One is with thiol-cleavable oxymethylene disulfide linker and the other is phosphine cleavable azidomethyl linker. Their synthesis involve multistep reactions and purification. The cleavable linkers are prepared first and then they are conjugated with amino-modified DNA probes. At the final step they were labeled by fluorescent dyes using the amino terminal of the cleavable linker.

### Synthesis of DCL-DNA probes with oxymethylene disulfide cleavable linker (5)

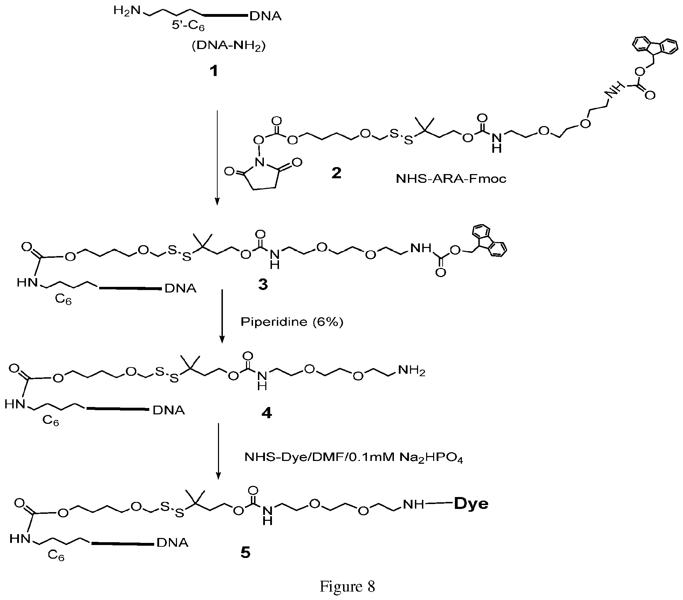

The synthesis steps are shown in figure 8. The activated linker 2 was obtained as described in US patents - Nucleotide analogues (Mong Marma, et al US 10,301,346, 2016) and Methods for synthesis of nucleotide analogues with disulfide linker (Mong Marma, et al, US 10,336,785, 2016). The synthesis involves following steps (Figure 8):

### (a) Linker installation and Fmoc group deprotection:

Amino modified DNA barcode(BC-NH₂,**1**) (~700 nmoles, source IDT DNA) was dissolved in 1.4 mL of DI water and transferred to 50 mL falcon tube. It was added with 0.3 mL of 0.5 M Na₂HPO₄ to make base concentration ~ 0.1 mM. In another 2.0 mL centrifuge tube, ∼ 6 mg of activated linker (NHS-ARA-Fmoc linker, compound **2**) was dissolved in 0.5 mL DMF. It was then added to the BC-NH₂ solution and stirred for 45 mins at room temperature. Once again, the same amount was added in 0.5 mL DMF, and stirred an additional 45 mins.

It was then quenched by adding 2.0 mL DI water. The mixture was then treated with 0.3 mL of pyperidin for 20 minutes. The product was immediately purified by prep-HPLC (X-bridge, 19X250 mm column, method: 0 - 3min 100%A, followed by 40% B over 40 min, then flat gradient to 50 mins). The target product usually eluted ∼ 43 mins. The target fraction was lyophilized and the solid product was suspended in 1.0 mL DI water, concentration was determined by UV at 260nm to yiled 160 nmole of compound **4** (1.0 mL × 0.160 mM).

### (b) Labeling with fluorescent dye:

The BC-ARA-NH₂ (compound **4**) obtained from earlier step was treated with Na₂HPO₄ salt (salt directly dissolved to avoid further dilution) to make 0.1 mM final concentration. It was then treated with ∼ 10 eq of NHS-Dye in 200 uL DI water (for low polar dye such as AF532-NHS, +100 uL DMF). The mixture was stirred for 30 minutes, and then immediately purified by prep-HPLC (X-bridge, 19×250 mm column, method: 0-3min 100%A, followed by 40% B over 40 min, the flat gradient to 50 mins). Target product **5** was eluted ~ 40 min. The target fraction was then lyophilized and the final product was dissolved in 1.0 mL RNAs free water and the concentration was determined by UV spectrum (based on dye's property). The product's concentration was then adjusted to prepare stock solution at 0.1 mM.

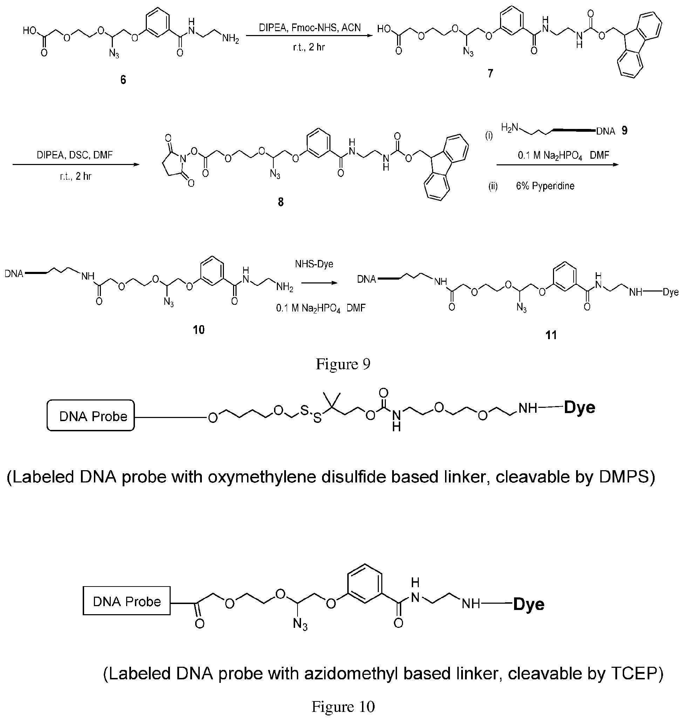

Synthesis of DCL-DNA probes with oxymethine-azide cleavable linker (11): The synthesis of DCL-DNA probes with -oxyazidomethine cleavable linker starts with a commercially available compound **6** as shown in figure 9. It involves following steps:
(a) Synthesis of Fmoc protected linker (**7**)
   (a) Activation of the linker (**9**)
   (b) Coupling to amino-modified DNA probes (**10**)
   (c) Coupling to fluorescent dye (**11**)

The synthesis processes for Fmoc protected linker (7) and the activation of the linker for coupling to DNA probes (9) are described below. The coupling reaction of the linker to DNA probe (10) and the final labeling reaction to produce the labeled probe (11) were carried as for oxymethyle disulfide described above.

To a solution of Fmoc-NHS (760 mg, 2.3 mmol, 1.2 eq) and compound 6 (734 mg, 1.9 mmol, 1 eq) in anhydrous DMF (12 mL), was added DIPEA (0.66 mL, 3.8 mmol, 2 eq). The resulting reaction mixture was stirred at room temperature for 2 hours. It was then diluted with EtOAc (20 mL) and quenched with 1M HCl (20 mL). The aqueous layer was separated and extracted with EtOAC (3x20 mL). The combined organic layers were washed with water (5x20 mL) and brine (sat., 20 mL), dried over Na2SO4 and concentrated under reduced pressure. The crude product was purified by flash chromatography (ISCO Silica 24 g Gold; Gradient: MeOH/DCM (0∼30%), 35 mL/min.) to give the desired product 7 as a white solid (488 mg, 44%). Next, to a solution of compound 7 (488 mg, 0.83 mmol, 1 eq) in anhydrous DMF (8.3 mL), was added DIPEA (0.22 mL, 1.3 mmol, 1.5 eq) and DSC (318 mg, 1.25 mmol, 1.5 eq). The resulting reaction mixture was stirred at room temperature for 2 hours. It was then concentrated under reduced pressure and the residue was purified by flash chromatography (ISCO Silica 24 g Gold; Gradient: EtOAc/hexane (30∼100%), 35 mL/min.) to give the desired product 8 as a syrup (408 mg, 72%).

### Staining experiments

Probes targeting four transcripts were designed with six unique hybridization domains. Two of the probes contained sequences that hybridize oligonucleotides with an azido linker (-OCHN3-) conjugated to Alexa Fluor 647, or oligonucleotides with a disulfide linker (-OCH2-SS-) conjugated to Alexa Fluor 532 (Figure 1A). To serve as a control, two of the probes contained two different binding regions that hybridized two oligonucleotides (Figure 1A). One probe contained sequences that hybridized both an oligonucleotide with an disulfide linker with Alexa Fluor 647 and an oligonucleotide an uncleavable Alex Fluor 532. The other probe had sequences that hybridized both an oligonucleotide with an azido linker with Alexa Fluor 532 and an oligonucleotide an uncleavable Alex Fluor 647. By having two independent fluorophores across the hybridization domains, we were able to evaluate the technology in multiple fluorescent wavelengths.

The probes used had the structure according to Figure 10.

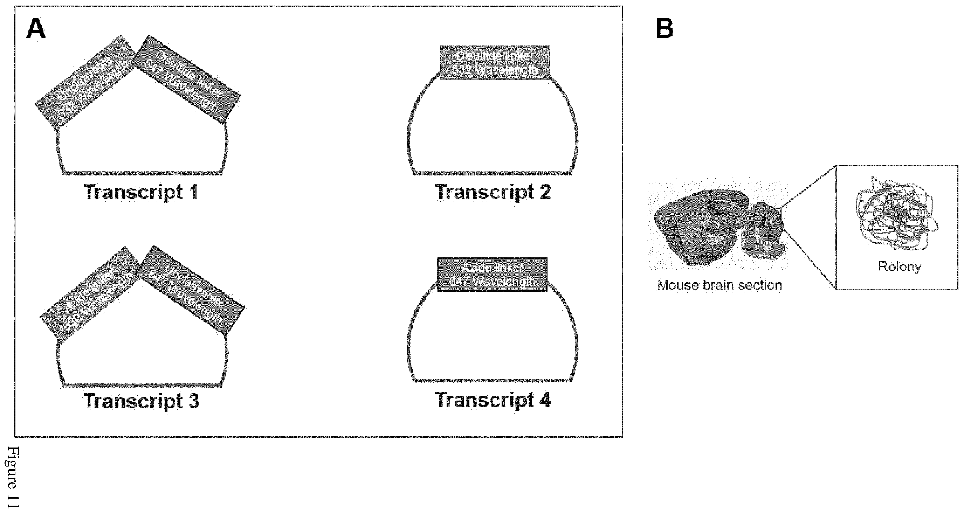

The probes were then hybridized on FFPE mouse brain sections, and then subsequently underwent rolling circle amplification to generate rolonies, or DNA nanoballs, which provided amplification to detect the signal *in situ* (Figure 11). All six complementary probes, or readout probes, were hybridized simultaneously after rolonies were generated.

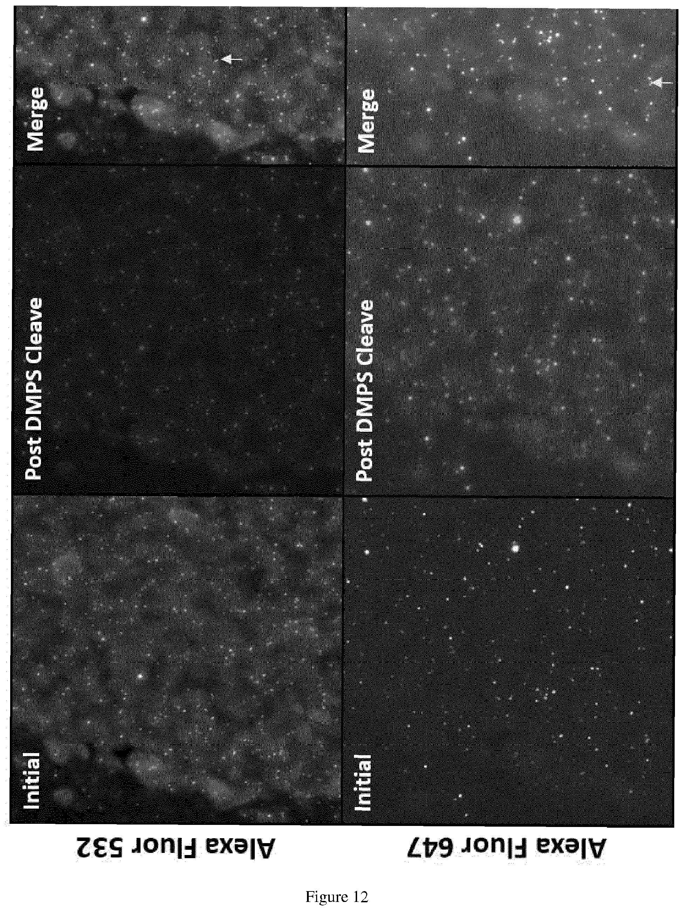

Next, a series of sequential chemical cleavage steps were performed. The first cleave step utilized Sodium 2,3-dimercaptopropanesulfonate monohydrate (DMPS), which selective cleaves the oligonucleotides containing the disulfide linker. Figure 12 shows the first of two serial cleavage rounds using DMPS to selective remove the fluorophores that were attached to the oligonucleotide via the azido linker. All images are pseudocolored to provide enough contrast to determine which puncta have signal removed between rounds. In the Alexa Fluor 532 wavelength, puncta that were cleaved by DMPS appear yellow in the merged image (yellow arrows). In the Alexa Fluor 647 wavelength, puncta that were cleaved by DMPS appear purple in the merged image. Objects that were not impacted by the DMPS cleave appear white in the merged image in both wavelengths.

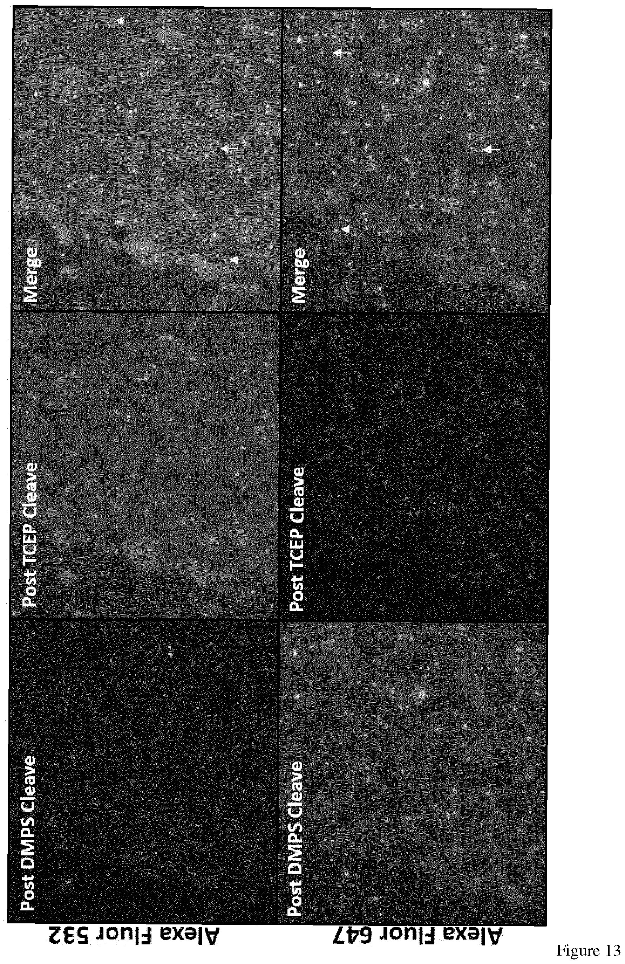

This was followed by a second cleavage using Tris(2-carboxyethyl)phosphine hydrochloride (TCEP) to cleave oligonucleotides containing the azido linker. Figure 13 shows the second of two serial cleavage rounds using TCEP to selective remove the fluorophores that were attached to the oligonucleotide via the disulfide linker. All images are pseudo-colored to provide enough contrast to determine which puncta have signal removed between rounds. In the Alexa Fluor 532 wavelength, puncta that were cleaved by TCEP appear purple (yellow arrows) in the merged image. In the Alexa Fluor 647 wavelength, puncta that were cleaved by TCEP appear blue in the merged image. After the TCEP cleavage, only non-cleavable oligonucleotides remain detectable and appear white in the merged image in both wavelengths.

After both cleave rounds, only the uncleavable puncta remained.

## Claims

1. Method for detecting RNA, DNA or protein target sequences by
a) Hybridizing a library of probes having the general formula (I)
P - (CL-D)ₓ (I)
With
P: probes having at least 10 nucleotides or amino acids
CL: cleavable linker
D: fluorescent dye
X: integer between 1 and 5
to RNA, DNA or protein target sequences wherein the library comprises probes P having different sequences of nucleotides or amino acids and cleavable linkers CL of different groups which are cleavable with different means
b) Removing unhybridized probes and detecting the hybridized probes *via* the fluorophores D by a first image
c) Cleaving sequentially by different means each group of chemical linkers CL from the hybridized probes; removing the thus cleaved fluorophores D and detecting the remaining hybridized probes via their fluorophores D by a second image
d) Detecting the removed fluorophores D by comparing the first and second image.
e) Obtaining a part of the sequence information of the target sequences via the sequence information of the probes P associated with the removed fluorophores D
f) Repeating step c) until all groups of chemical linkers CL are cleaved.

2. Method according to claim 1 **characterized in that** after step f), the probes P are de-hybridized from the RNA, DNA or protein target sequences.

3. Method according to claim 1 or 2 characterized that step a) to f) are repeated until the sequence information of the RNA, DNA or protein target sequences is obtained.

4. Method according to any of the claims 1 to 3 characterized that the cleavable linker CL is cleavable by chemical, photochemical or enzymatical means.

5. Method according to any of the claims 1 to 4 characterized that the cleavable linker CL is an enzymatically cleavable linker selected from the group consisting of polysaccharides, proteins, peptides, depsipeptides, polyesters, nucleic acids, and derivatives thereof.

6. Method according to claim 5 characterized that the polysaccharides are selected from the group consisting of dextrans, pullulans, inulins, amylose, cellulose, hemicelluloses, xylan, glucomannan, pectin, chitosan, and chitin.

7. Method according to any of the claims 1 to 4 characterized that the cleavable linker CL is selected from the group consisting of disulfide linkers (-SS-, (a)), oxymethylene disulfides (-OCH₂SSCR₁R₂-, (b)), oxymethine-azides (-CR₁,R₂OCH(N₃)R₃,R₄)-, (c)), azo-arenes (-ArN=NAr-, (d)), nitrobenzyl derivatives - (e), allyl derivatives (f), thiocarbamate (g), where R₁, R₂, R₃, R₄ can be independently H, methyl, ethyl, propyl, t-butyl, C5-C10 alkyls, alkenes, alkynes, hydroxyl, halogens, amines, amides, carboxylates, polyethylene glycols.

8. Method according to any of the claims 1 to 7 characterized that the library comprises probes having at least two different groups of cleavable linkers CL which are cleavable with two different means selected from the group consisting of two different photochemical activation radiations, two different enzymes, two different chemical agents, one photochemical activation radiation and one enzyme, one photochemical activation radiation and one chemical agent, one enzyme and one chemical agent.
